# EUROPEAN PATENT SPECIFICATION

(11) **EP 1 532 168 B1**
(45) Date of publication and mention of the grant of the patent: **23.07.2014**
(21) Application number: 03757819.2
(22) Date of filing: 28.08.2003
(51) Int. Cl.: C07K 14/22, A61K 39/095

(54) **METHOD FOR REFOLDING NEISSERIAL NSPA PROTEIN**
METHODE ZUR RÜCKFALTUNG VON NSPA PROTEIN VON NEISSERIA
PROCEDE DE REPLIEMENT

(30) Priority: 30.08.2002 GB 0220197
(43) Date of publication of application: 25.05.2005
(73) Proprietor: GlaxoSmithKline Biologicals s.a., 1330 Rixensart (BE); Utrecht University, 3584 CH Utrecht (NL)
(72) Inventor: Biemans, Ralph, 1330 Rixensart (BE); Bos, Martine, 3584 CH Utrecht (NL); Denoel, Philippe, 1330 Rixensart (BE); Feron, Christiane, 1330 Rixensart (BE); Goraj, Carine, 1330 Rixensart (BE); Poolman, Jan, 1330 Rixensart (BE); Tommassen, Jan, 3584 CH Utrecht (NL); Weynants, Vincent, 1330 Rixensart (BE)
(74) Representative: Dalton, Marcus Jonathan William
(86) International application number: PCT/EP2003/010085
(87) International publication number: WO 2004/020452

(56) References cited:
- WO-A-00/71725
- WO-A-01/09350
- WO-A-01/72337
- WO-A-96/29412
- JANSEN C ET AL: "Biochemical and biophysical characterization of in vitro folded outer membrane porin PorA of Neisseria meningitidis" BIOCHIMICA ET BIOPHYSICA ACTA. BIOMEMBRANES, AMSTERDAM, NL, vol. 1464, no. 2, 5 April 2000 (2000-04-05), pages 284-298, XP004273195 ISSN: 0005-2736
- KRAMER R A ET AL: "In vitro folding, purification and characterization of Escherichia coli outer membrane protease OmpT" EUROPEAN JOURNAL OF BIOCHEMISTRY, vol. 267, no. 3, February 2000 (2000-02), pages 885-893, XP001189705 ISSN: 0014-2956
- DEKKER NIEK ET AL: "In vitro folding of Escherichia coli outer-membrane phospholipase A" EUROPEAN JOURNAL OF BIOCHEMISTRY, vol. 232, no. 1, 1995, pages 214-219, XP008029422 ISSN: 0014-2956
- MOE G R ET AL: "Differences in surface expression of NspA among Neisseria meningitidis group B strains" INFECTION AND IMMUNITY, AMERICAN SOCIETY FOR MICROBIOLOGY. WASHINGTON, US, vol. 67, no. 11, November 1999 (1999-11), pages 5664-5675, XP002266209 ISSN: 0019-9567
- VANDEPUTTE-RUTTEN LUCY ET AL: "Crystal structure of neisserial surface protein A (NspA), a conserved outer membrane protein with vaccine potential." JOURNAL OF BIOLOGICAL CHEMISTRY, vol. 278, no. 27, 4 July 2003 (2003-07-04), pages 24825-24830, XP002274972 ISSN: 0021-9258

## Description

### Field of the Invention

The present invention relates to a method of refolding NspA protein - an outer membrane protein of *Neisseria meningitidis* organisms, and the disclosure relates to such refolded proteins, pharmaceutical compositions comprising them, and their use in the treatment, prevention and diagnosis of bacterial infections, such as Neisserial infections, and particularly, but not exclusively, *Neisseria meningitidis* and/or *Neisseria gonorrhoeae.*

### Background of the Invention

Neisserial strains of bacteria are the causative agents for a number of human pathologies, against which there is a need for effective vaccines to be developed. In particular *Neisseria gonorrhoeae* and *Neisseria meningitidis* cause pathologies which could be treated by vaccination.

*Neisseria gonorrhoeae* is the etiologic agent of gonorrhea, one of the most frequently reported sexually transmitted diseases in the world with an estimated annual incidence of 62 million cases (Gerbase et al 1998 Lancet 351; (Suppl 3) 2-4). The clinical manifestations of gonorrhea include inflammation of the mucus membranes of the urogenital tract, throat or rectum and neonatal eye infections. Ascending gonococcal infections in women can lead to infertility, ectopic pregnancy, chronic pelvic inflammatory disease and tubo-ovarian abscess formation. Septicemia, arthritis, endocarditis and meningitis are associated with complicated gonorrhea.

The high number of gonococcal strains with resistance to antibiotics contributes to increased morbidity and complications associated with gonorrhea. An attractive alternative to treatment of gonorrhea with antibiotics would be its prevention using vaccination. No vaccine currently exists for *N*. *gonorrhoeae* infections.

*Neisseria meningitidis* (meningococcus) is a Gram-negative bacterium frequently isolated from the human upper respiratory tract. It occasionally causes invasive bacterial diseases such as bacteremia and meningitis. Most cases of disease are in infants or young children. The incidence of meningococcal disease shows geographical seasonal and annual differences (Schwartz, B., Moore, P.S., Broome, C.V.; Clin. Microbiol. Rev. 2 (Supplement), S18-S24, 1989). Most disease in temperate countries is due to strains of serogroup B and varies in incidence from 1-10/100,000/year total population sometimes reaching higher values (Kaczmarski, E.B. (1997), Commun. Dis. Rep. Rev. 7: R55-9, 1995; Scholten, R.J.P.M., Bijlmer, H.A., Poolman, J.T. et al. Clin. Infect Dis. 16: 237-246, 1993; Cruz, C., Pavez, G., Aguilar, E., et al. Epidemiol. Infect. 105: 119-126, 1990).

Epidemics dominated by serogroup A meningococci, mostly in central Africa, are encountered, sometimes reaching levels up to 1000/100.000/year (Schwartz, B., Moore, P.S., Broome, C.V. Clin. Microbiol. Rev. 2 (Supplement), S18-S24, 1989). Nearly all cases as a whole of meningococcal disease are caused by serogroup A, B, C, W-135 and Y meningococci and a tetravalent A, C, W-135, Y polysaccharide vaccine is available (Armand, J., Arminjon, F., Mynard, M.C., Lafaix, C., J. Biol. Stand. 10: 335-339, 1982).

The frequency of *Neisseria meningitidis* infections has risen dramatically in the past few decades. This has been attributed to the emergence of multiply antibiotic resistant strains and an increasing population of people with weakened immune systems. It is no longer uncommon to isolate *Neisseria meningitidis* strains that are resistant to some or all of the standard antibiotics. This phenomenon has created an unmet medical need and demand for new anti-microbial agents, vaccines, drug screening methods, and diagnostic tests for this organism.

Martin D et al Journal of Biotechnology 83 (2000) 27-31 reports that there is presently no effective vaccine that can stimulate protective group-common immunity in young children. Efforts are being made to improve the current polysaccharide vaccines through conjugation to carrier proteins or to find other meningococcal surface antigens that could become the basis of a protein vaccine. However, the interstrain variability of the major outer membrane proteins would restrict their protective efficacy to a limited number of antigenically related strains. Neisseria surface protein A (referred to herein as NspA) has characteristics which indicate that it is a potential vaccine candidate for the development of a group-common vaccine against meningocococcal disease.

It is envisaged that recombinant NspA expressed in cells could be produced for use in such new anti-microbial agents, vaccines, drug screening methods, and diagnostic tests. However, one of the major limitations on the expression of proteins is the inability of many recombinant proteins to fold into their biologically active conformations. Often only low yields of the recombinant protein are obtained due to aggregation and mis-folding of the unfolded species. Indeed, protein refolding, in which the protein acquires it native and active structure, is one of the biggest challenges in molecular biology. Jansen et al. (Biochim. et Biophys. Acta. Biomembranes, 2000 1464:284-298) describe the characterization of in vitro folded PorA of *Neisseria* menigitidis.

Given the problems associated with obtaining biologically active refolded recombinant proteins, the use of non-live vectors, for example bacterial outer-membrane vesicles or "blebs" has been envisaged. OM blebs are derived from the outer membrane of the two-layer membrane of Gram-negative bacteria and have been documented in many Gram-negative bacteria (Zhou, L et al. 1998. FEMS Microbiol. Lett. 163:223-228). However, blebs have the disadvantage that they may express outer-membrane proteins which are either not relevant (e.g. unprotective antigens or immunodominant but variable proteins) or detrimental (e.g. toxic molecules such as LPS, or potential inducers of an autoimmune response). The need therefore remains to provide a subunit vaccine against Neisseria disease comprising purified protective outer membrane proteins in a refolded conformation suitable to elicit an effective immune response.

### Summary of the Invention

The present invention provides an improved method for refolding the NspA protein. We have now shown that it is possible to increase the recovery of active protein from partly purified inclusion bodies in amounts up to 100%, without the need for further purification.

The present disclosure relates to refolded NspA protein and methods for using such proteins, including prevention and treatment of microbial diseases, amongst others in e.g. subunit vaccines. In a further aspect, the disclosure relates to diagnostic assays for detecting diseases associated with microbial infections and conditions associated with such infections.

### Statements of the Invention

According to one aspect of the present invention there is provided a method for refolding NspA protein comprising contacting the NspA with an alkaline refolding buffer comprising 3-dimethyldodecylammoniopropanesulfonate (hereinafter referred to as SB-12).

Preferably the refolding buffer comprises ethanolamine and SB-12.

Preferably the refolding buffer has pH11.

Preferably the SB-12 is 0.2-1% or 0.3-0.8% SB-12.

Preferably the SB-12 is 0.2% SB-12.

In another preferred embodiment the SB-12 is 0.5% SB-12.

In further preferred embodiment the SB-12 is 1% SB-12.

Preferably the SB-12 is purified.

Preferably the SB-12 is purified by passing it over an Al₂O₃ column.

Preferably the ethanolamine is about 20mM ethanolamine (most preferably about pH11).

According to another aspect of the present invention there is provided a method comprising the following steps:
expressing NspA in a host cell;
breaking the host cell to obtain an inclusion body comprising NspA;
washing the inclusion body;
solubilisation of NspA and/or the inclusion body;
contacting the solubilised NspA with the refolding buffer of the invention; and
removing the refolding buffer from the NspA.

According to an aspect of the disclosure there is provided a refolding buffer comprising ethanolamine and SB-12 (for instance in the concentrations mentioned above) for use in the method of the present invention.

According to an aspect of the present disclosure there is provide an isolated, refolded NspA protein obtained or obtainable by the method of the present invention.

According to another aspect of the present disclosure there is provided a pharmaceutical composition comprising at least one isolated, refolded NspA protein of the present disclosure, and a pharmaceutically acceptable carrier.

Preferably at least 30%, 50%, 70%, or 90% of the NspA protein present in the composition of the disclosure is refolded.

In one embodiment of the disclosure the pharmaceutical composition is in the form of a vaccine.

Preferably said composition comprises at least one other Neisserial antigen.

Preferably said composition further comprises at least one other Neisserial adhesin, or one other Neisserial outer membrane protein, or at least one Neisserial autotransporter, or at least one Neisserial toxin, or at least one Neisserial Fe acquisition protein. Most preferably, in addition to isolated, refolded NspA, at least one Neisserial autotransporter and at least one Neisserial toxin is present, or at least one Neisserial autotransporter and at least one Neisserial Fe acquisition protein is present, or at least one Neisserial toxin and at least one Neisserial Fe acquisition protein is present, or at least one Neisserial autotransporter and at least one Neisserial Fe acquisition protein and at least one Neisserial toxin is present, or immunogenic fragments thereof.

More preferably the pharmaceutical composition of the disclosure comprises at least one further antigen (or fragment thereof) selected from at least one of the following classes:
at least one Neisserial adhesin selected from the group consisting of FhaB, Hsf,NadA, PilC, Hap, MafA, MafB, Omp26, NMB0315, NMB0995 and NMB 1119;
at least one Neisserial autotransporter selected from the group consisting of Hsf, Hap, IgA protease, AspA and NadA;
at least one Neisserial toxin selected from the group consisting of FrpA, FrpC, FrpA/C, VapD, NM-ADPRT, and either or both of LPS immunotype L2 and LPS immunotype L3;
at least one Neisserial Fe acquisition protein selected from the group consisting of TbpA high, TbpA low, TbpB high, TbpB low, LbpA, LbpB, P2086, HpuA, HpuB, Lipo28, Sibp, FbpA, BfrA, BfrB, Bcp, NMB0964 and NMB0293; and
at least one Neisserial membrane associated protein, preferably outer membrane protein, selected from the group consisting of PldA, TspA, FhaC, NspA, TbpA(high), TbpA(low), LbpA, HpuB, TdfH, PorB, HimD, HisD, GNA1870, OstA, HlpA, MltA, NMB 1124, NMB 1162, NMB 1220, NMB 1313, NMB 1953, HtrA, TspB, PilQ and OMP85.

Most preferably the pharmaceutical composition of the disclosure comprises at least one further antigen (or fragment thereof) selected from at least one of the following classes:
at least one Neisserial adhesin selected from the group consisting of FhaB, Hsf and NadA;
at least one Neisserial autotransporter selected from the group consisting of Hsf, and Hap;
at least one Neisserial toxin selected from the group consisting of FrpA, FrpC, and either or both of LPS immunotype L2 and LPS immunotype L3;
at least one Neisserial Fe acquisition protein selected from the group consisting of TbpA, TbpB, LbpA and LbpB; and
at least one Neisserial outer membrane protein (or fragment thereof) selected from the group consisting of PldA, TspA, TspB, PilQ and OMP85.

Preferably the pharmaceutical composition of the present disclosure comprises a further antigen derived from *Neisseria meningitidis.*

Preferably the pharmaceutical composition of the present disclosure further comprises an antigen derived from *Neisseria gonorrhoeae.*

Preferably the pharmaceutical composition of the present disclosure is at least in part a subunit preparation. Although it may be a subunit preparation mixed with a bleb preparation comprising additional Neisserial antigens of the disclosure (preferably upregulated in expression), it is also preferred that the pharmaceutical compositions of the present disclosure is entirely a subunit preparation with any additional Neisserial antigens of the disclosure being present in a refolded form, or as soluble surface-exposed fragment of the additional Neisserial antigen.

Preferably the pharmaceutical composition of the disclosure further comprises bacterial capsular polysaccharides or oligosaccharides.

Preferably said capsular polysaccharides or oligosaccharides are derived from bacteria selected from the group consisting of *Neisseria meningitidis* serogroup A, C, Y, and/or W-135, *Haemophilus influenzae b, Streptococcus pneumoniae,* Group A Streptococci, Group B Streptococci, *Staphylococcus aureus* and *Staphylococcus epidermidis,* and are most preferably conjugated to a source ofT-helper epitopes.

According to another aspect of the present disclosure there is provided an antibody immunospecific for the NspA protein of the present disclosure.

According to another aspect of the present disclosure there is provided a method of diagnosing a Neisserial infection, comprising identifying an NspA protein of the present disclosure, or an antibody that is immunospecific for said protein, present within a biological sample from an animal, including a human, suspected of having such an infection, or by using an NspA protein or antibody of the present disclosure to detect whether NspA or antibodies against NspA are present within a biological sample from an animal.

Preferably the method relates to diagnosis of *Neisseria meningitidis* and most preferably *Neisseria meningitidis* serogroup B.

In another preferred embodiment of the disclosure the method relates to diagnosis of *Neisseria gonorrhoeae.*

According to another aspect of the present disclosure there is provided use of a composition comprising an NspA protein of the present invention in the preparation of a medicament for use in generating an immune response in an animal.

In one embodiment of the disclosure the use of the vaccine is in the preparation of a medicament for treatment or prevention of Neisserial infection.

In one preferred embodiment of the disclosure the use is in the preparation of a medicament for treatment or prevention of *Neisseria meningitidis* infection and most preferably *Neisseria meningitidis* serogroup B.

In another preferred embodiment of the disclosure the use is in the preparation of a medicament for the treatment or prevention of *Neisseria gonorrhoeae* infection.

According to yet another aspect of the present disclosure there is provided a pharmaceutical composition useful in treating humans with a Neisserial disease comprising at least one antibody directed against the NspA protein of the present disclosure and a suitable pharmaceutical carrier.

According to a further aspect of the present disclosure there is provided use of the antibody of the present disclosure in the manufacture of a medicament for the treatment or prevention of Neisserial disease.

According to one preferred embodiment of the disclosure *Neisseria meningitidis* infection is prevented or treated and most preferably *Neisseria meningitidis* serogroup B infection.

According to another preferred embodiment of the disclosure *Neisseria gonorrhoeae* infection is prevented or treated.

### Detailed Description

Various preferred features and embodiments of the present invention will now be described by way of non-limiting example with reference to the accompanying drawing in which:

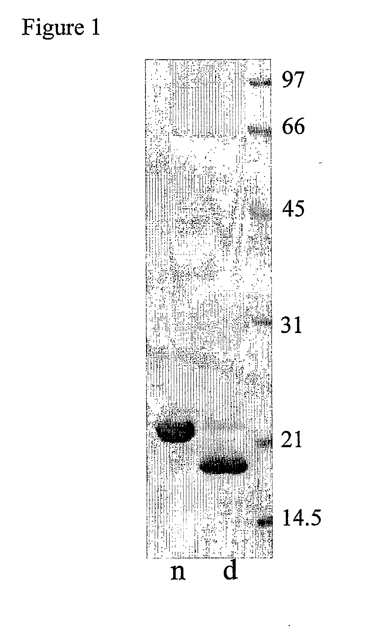

Figure 1 shows an SDS-PAGE gel comparing NspA refolded according to the method of the present invention and denatured NspA. In more detail, Figure 1 shows the heat-modifiability of purified refolded NspA in Coomassie Blue stained 14% PAGE. n = purified refolded NspA run in semi-native conditions, and d = purified refolded NspA run in denaturing conditions. The molecular weight markers are shown on the night.

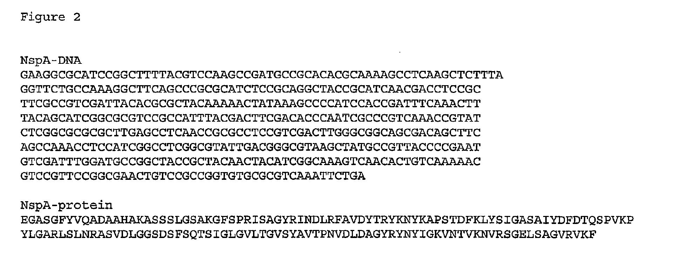

Figure 2 shows the nucleotide and amino acid sequence of the H44/76 NspA used in the Examples; and

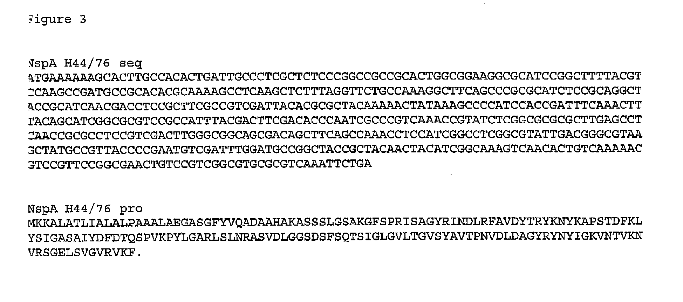

Figure 3 shows the nucleotide and amino acid of NspA including leader sequence.

Although in general the techniques mentioned herein are well known in the art, reference may be made in particular to Sambrook et al., Molecular Cloning, A Laboratory Manual (1989) and Ausubel et al., Short Protocols in Molecular Biology (1999) 4th Ed, John Wiley & Sons, Inc (as well as the complete version Current Protocols in Molecular Biology).

The terms "comprising", "comprise" and "comprises" herein is intended by the inventors to be optionally substitutable with the terms "consisting of", "consist of", and "consists of", respectively, in every instance.

### Method

The present invention provides a method for promoting the correct folding/refolding of an NspA protein which method involves the use of the detergent SB-12 in an alkaline refolding buffer.

Typically the method of the present invention is used to assist in refolding recombinantly produced NspA, which is obtained in an unfolded or misfolded form. Thus, recombinantly produced proteins may be contacted with the refolding buffer to unfold, refold and/or reactivate recombinant proteins which are inactive due to misfolding and/or are unfolded as a result of their extraction from the host cells in which they were expressed (such as from bacterial inclusion bodies). Such a process may also be termed "reconditioning".

The method of the invention may be employed to maintain the folded conformation of NspA, for example during storage, in order to increase shelf life. Under storage conditions, many proteins lose their activity, as a result of disruption of correct folding. The presence of the refolding buffer of the present invention, reduces or reverses the tendency of proteins to become unfolded and thus greatly increases the shelf life thereof.

The method of the invention may be used to promote the correct folding of NspA which, through storage, exposure to denaturing condition or otherwise, have become misfolded. Thus, the invention may be used to recondition NspA. For example, NspA in need of reconditioning may be contacted with the refolding buffer in accordance with the invention.

The present invention also provides a method for altering the structure of an NspA protein. Structural alterations include folding, unfolding and refolding. The effect of the alterations is preferably to improve the yield, specific activity and/or quality of the molecule. This may typically be achieved by resolubilising, reconditioning and/or reactivating incorrectly folded molecules post-synthesis.

The terms "reconditioning" and "reactivating" thus encompass *in vitro* procedures. Particular examples of *in vitro* procedures may include processing proteins that have been solubilised from cell extracts (such as inclusion bodies) using strong denaturants such as urea or guanidium chloride.

The terms "refold", "reactivate" and "recondition" are not intended as being mutually exclusive. For example, an inactive protein, perhaps denatured using urea, may have an unfolded structure. This inactive protein may then be refolded with a refolding buffer of the invention thereby reactivating it. In some circumstances there may be an increase in the specific activity of the refolded/reactivated protein compared to the protein prior to inactivation/denaturation: this is termed "reconditioning".

The molecule is typically an unfolded or misfolded protein which is in need of folding. Alternatively, however, it may be a folded protein which is to be maintained in a folded state.

The invention envisages at least two situations. A first situation is one in which the protein to be folded is in an unfolded or misfolded state, or both. In this case, its correct folding is promoted by the method of the invention. A second situation is one in which the protein is substantially already in its correctly folded state, that is all or most of it is folded correctly or nearly correctly. In this case, the method of the invention serves to maintain the folded state of the protein by affecting the folded/unfolded equilibrium so as to favour the folded state. This prevents loss of activity of an already substantially correctly folded protein. These, and other, eventualities are covered by the reference to "promoting" the folding of the protein.

As used herein, a protein may be unfolded when at least part of it has not yet acquired its correct or desired secondary or tertiary structure. A protein is misfolded when it has acquired at least partially incorrect or undesired secondary or tertiary structure. Techniques are known in the art for assessing protein structure - such as circular dichroism.

The refolding buffer of the present invention comprises 3-dimethyldodecylammoniopropanesulfonate (also referred to as N-Dodecyl-N,N-dimethyl-3-ammonio-1-propanesulfonate) and referred to herein as "SB-12". SB-12 is available from commercial sources, such as Fluka AG. Whilst not wishing to be bound by any theory, we believe the dilution step in SB-12 (or the presence of SB-12 in general) creates a hydrophobic environment for the protein, which is similar to the protein's natural *in vivo* environment.

SB-12 is a detergent and the concentration of SB-12 should be at least about 0.2% (w/v), since this is the concentration generally required for micelle formation. Thus, the concentration of SB-12 may be about 0.2% to about 5.0%, about 0.3% to about 4.0%, about 0.4% to about 3.0%, or about 0.5% to about 2.0%. Preferably the concentration is about 0.2%, 0.3%, 0.4%, 0.5%, 0.6%, 0.7%, 0.8%, 0.9% or 1.0%. In an especially preferred embodiment 0.5% SB-12 is used. By "about" it is preferably meant +/-10% of the value of the figure quoted, but is most preferably the exact figure quoted

In a preferred embodiment the SB-12 is purified. We have found improved folding with purified SB-12 compared to non-purified SB-12. Conveniently the SB-12 may be purified before use by passing a concentrated solution of SB-12 over an Al₂O₃ chromatography column, e.g. using in methanol/chloroform (1:1), but any suitable method may be used for purifying the SB-12.

We have found that the dilution of the denatured protein should to be carried out in an alkaline environment to maximise the efficiency of refolding. Preferably the pH of the refolding buffer is about 11.0. Preferably the alkaline environment is obtained by the use of ethanolamine. In this preferred embodiment the refolding buffer comprises SB-12 and ethanolamine. Conveniently 20mM ethanolamine is used, but other concentrations, such as 50mM, may be useful.

We have found that a 1:20 dilution of the NspA in the refolding buffer is preferred, but other ratios such as 1:10 may be used. Five-fold dilution into refolding buffer may also be used.

The NspA to be processed by the method of the invention is typically obtained from cell extracts of host cells expressing recombinant NspA. Host cells include prokaryotes such as *E*. *coli,* yeast and insect cells (the baculovirus system is capable of very high level protein expression). Expression of the NspA in the host cell is preferably at high levels to maximise yield. Further details on the expression of recombinant NspA are given below.

We have found that the present invention is most efficient when used to refold a mature NspA protein, i.e. a protein without a leader or secretory sequence, a pre-, or pro- or prepro-protein sequence, and also a protein. Typically, for NspA, this means expressing the protein without the signal peptide (amino acids 1-19). It is common during conventional purification techniques to make use of a marker sequence that facilitates purification, such as a hexa-histidine peptide, as provided in the pQE vector (Qiagen, Inc.) and described in Gentz et al., Proc. Natl. Acad. Sci., USA 86: 821-824 (1989), or an HA peptide tag (Wilson et al., Cell 37: 767 (1984). We have found that the process of the present invention is most efficient when such marker sequences are not present.

It is likely that a substantial proportion of the NspA will be insoluble and consequently techniques to solubilise normally insoluble components of the cell extracts (such as inclusion bodies) to maximise extraction of the NspA will typically be employed. Any conventional technique for preparation and extraction of the NspA proteins from inclusion bodies and their subsequent solubilisation may be employed. Such techniques are described for example in "Current Protocols in Protein Science" published by JA Wiley & Sons. Such techniques generally include:
Cell Lysis - using, for example, a French press or sonication, to release inclusion bodies. Typically the cells are placed in a cold buffer such as a TE buffer prior to lysis. Sonication may be carried out using a Branson sonifier. Sonification may take place in the presence of a detergent, e.g. Brij or Triton. The inclusion bodies in the cell lysate may then be pelleted using low-speed centrifugation. The cells may be pretreated with lysozyme prior to lysis. The purpose of the pretreatment is to aid removal of the peptidoglycan and outer membrane protein contaminants during the washing steps. The lysed cells may be clarified by centrifugation and the supernatant discarded.

Inclusion Body Washing - to remove cell wall and other outer membrane components contaminants from the inclusion bodies recovered from cell lysates. Typically the pellet is resuspended in a wash buffer containing, e.g. buffer such as TE buffer and/or a detergent such as Triton. The suspension may then be resuspended and the supernatant discarded. This process may be repeated until the supernatant is clear. If required, the washed pellets can be frozen for storage.

The amount of recombinant protein in the washed pellet may be estimated using the following guidelines: (1) an expression level of 1% corresponds to ∼1mg recombinant protein per 1 g wet cells. (2) The recovery of highly aggregated recombinant protein in the washed pellets is ∼75% that originally present in the cells. (3) About 60% of the total washed pellet protein is recombinant-derived. The total amount of recombinant protein can be directly determined be measuring the total protein concentration or by analysing the washed pellets via SDS-PAGE to determine the proportions of the protein constituents.

Protein solubilisation - the extracted protein is then extracted from the washed pellet and defolded using a denaturant which disassociates protein-protein interactions and unfolds the protein so that it consists of unfolded monomers. Denaturants include guanidine:HCl (such as 6M guanidine:HCl) and/or urea (such as 8M urea). Residual insoluble material and materials can be typically removed by ultracentrifugation (e.g. 100,000 g x for 1 hr.). The extract may be stored by freezing following pelleting.

Solubilised cell extracts may optionally be partially purified by, for example, a variety of affinity chromatography techniques prior to contacting with the refolding buffer according to the method of the invention.

The solubilised cell extract is then diluted into the refolding buffer in accordance with the present invention. It is typically, but not necessarily, left stirring at room temperature overnight.

Thus, the starting material for the refolding/reconditioning method of the invention is typically denatured proteins in solutions of agents such as urea/guanidium chloride. Alternatively, or in addition, soluble protein samples may be specifically denatured by the addition of appropriate denaturing agents prior to refolding.

The method of the invention may also employ the use of molecular chaperones. Chaperones, including chaperonins, are proteins which promote protein folding by non-enzymatic means, in that they do not catalyse the chemical modification of any structures in folding proteins, but promote the correct folding of proteins by facilitating correct structural alignment thereof. Molecular chaperones are well known in the art, several families thereof being characterised. The invention may employ any molecular chaperone molecule, which term includes, for example, the molecular chaperones selected from the following non-exhaustive group:
p90 Calnexin, HSP family, HSP70 family, DNA K, DNAJ, HSP60 family (GroEL), ER-associated chaperones, HSP90, Hsc70, sHsps; SecA; SecB, Trigger factor, zebrafish hsp 47, 70 and 90, HSP 47, GRP 94, Cpn 10, BiP, GRP 78, C1p, FtsH, Ig invariant chain, mitochondrial hsp70, EBP, mitochondrial m-AAA, Yeast Ydj1, Hsp104, ApoE, Syc, Hip, TriC family, CCT, PapD and calmodulin (see WO99/05163 for references).

The method of the present invention may also make use of a foldase. In general terms, a foldase is an enzyme which participates in the promotion of protein folding through its enzymatic activity to catalyse the rearrangement or isomerisation of bonds in the folding protein. They are thus distinct from a molecular chaperone, which bind to proteins in unstable or non-native structural states and promote correct folding without enzymatic catalysis of bond rearrangement. Many classes of foldase are known, and they are common to animals, plants and bacteria. They include peptidyl prolyl isomerases and thiol/disulphide oxidoreductases. The invention may employ any of the foldases which are capable of promoting protein folding through covalent bond rearrangement.

At the end of the refolding/reconditioning process, the refolded NspA may be desalted by dialysis against a suitable storage buffer and/or the use of a desalting column into a suitable storage buffer. Suitable buffers include 25 mM sodium phosphate, 150 mM NaCl and 0.1% PEG 6000 (pH 7.4).

### Vectors, Host Cells, Expression Systems

The invention may employ vectors that comprise a polynucleotide which codes for at least an NspA protein, host cells that are genetically engineered with vectors of the invention and the production of NspA proteins by recombinant techniques. Cell-free translation systems can also be employed to produce such proteins using RNAs derived from DNA constructs.

Recombinant proteins of the present disclosure may be prepared by processes well known to those skilled in the art from genetically engineered host cells comprising expression systems.

For recombinant production of the proteins of the disclosure, host cells can be genetically engineered to incorporate expression systems or portions thereof or polynucleotides of the disclosure. Introduction of a polynucleotide into the host cell can be effected by methods described in many standard laboratory manuals, such as Davis, et al., BASIC METHODS IN MOLECULAR BIOLOGY, (1986) and Sambrook, et al., MOLECULAR CLONING: A LABORATORY MANUAL, 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, transduction, scrape loading, ballistic introduction and infection.

Representative examples of appropriate hosts include bacterial cells, such as cells of streptococci, staphylococci, enterococci, *E*. *coli,* streptomyces, cyanobacteria, *Bacillus subtilis, Moraxella catarrhalis, Haemophilus influenzae* and *Neisseria meningitidis;* fungal cells, such as cells of a yeast, *Kluveromyces, Saccharomyces,* a basidiomycete, *Candida albicans* and *Aspergillus*; insect cells such as cells of *Drosophilia* S2 and *Spodoptera* Sf9; animal cells such as CHO, COS, HeLa, C127, 3T3, BHK, 293, CV-1 and Bowes melanoma cells; and plant cells, such as cells of a gymnosperm or angiosperm.

A great variety of expression systems can be used to produce the proteins of the disclosure. Such vectors include, among others, chromosomal-, episomal- and virus-derived vectors, for example, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses, picornaviruses, retroviruses, and alphaviruses and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a protein in a host may be used for expression in this regard. The appropriate DNA sequence may be inserted into the expression system by any of a variety of well-known and routine techniques, such as, for example, those set forth in Sambrook et al., MOLECULE CLONING, A LABORATORY MANUAL*,* (*supra*).

In recombinant expression systems in eukaryotes, for secretion of a translated protein into the lumen of the endoplasmic reticulum, into the periplasmic space or into the extracellular environment, appropriate secretion signals may be incorporated into the expressed protein. These signals may be endogenous to the protein or they may be heterologous signals.

Proteins of the present disclosure can be recovered and purified from recombinant cell cultures by the method of the present invention.

### NspA protein

The present disclosure provides an isolated, refolded NspA protein. As used herein the term "protein" includes the term "polypeptide". By "isolated" we mean that the NspA protein is free from other proteins with which it is normal associated The NspA protein may be a recombinant protein. By "recombinant" we mean that the protein has been obtained using the application of molecular biology. However the method is also applicable to natural or synthetic proteins which require refolding, or purification by means of unfolding and refolding. Preferably the isolated, refolded NspA of the disclosure is purified in that it is more than 40, 50, 60, 70, 80, 90, 95, or 99% pure. Most preferably the NspA of the disclosure is biologically pure in that it is more than 40, 50, 60, 70, 80, 90, 95, or 99% free of other Neisserial proteins and/or other proteins of the host cell from which it was made.

Where a protein is specifically mentioned herein, it is preferably a reference to a native, full-length protein but it may also encompass antigenic fragments thereof (particularly in the context of subunit vaccines). These are fragments which contain or comprise at least 10 amino acids, preferably 20 amino acids, more preferably 30 amino acids, more preferably 40 amino acids or most preferably 50 amino acids, taken contiguously from the amino acid sequence of the protein. In addition, antigenic fragments denotes fragments that are immunologically reactive with antibodies generated against the Neisserial proteins or with antibodies generated by infection of a mammalian host with Neisseria. Antigenic fragments also includes fragments that when administered at an effective dose, elicit a protective immune response against Neisserial infection, more preferably it is protective against *N. meningitidis* and/or *N. gonorrhoea* infection, most preferably it is protective against *N. meningitidis* serogroup B infection.

The present disclosure also includes variants of the proteins mentioned herein, that is proteins that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, 1-3, 1-2 or 1 amino acids are substituted, deleted, or added in any combination.

It has been reported that the highly conserved NspA protein has been found in the outer membrane of every *Neisseria meningitidis* strain tested so far (Martin D et al Journal of Biotechnology 83 (2000) 27-31). Martin D et al also show that the NspA protein is exposed at the surface of intact meningococcal strains. The NspA protein has also been identified in *Neisseria gonorrhoeae* (Plante et al Infect. Immun. 67 (1999) 2855-2861). The meningococcal *nspA* gene may be amplified directly by PCR from chromosomal DNA (Martin D et al & Plante et al 1999 *supra).* NspA is also described in WO96/29412.

The NspA produced by the methods of the present invention, fragment or variant thereof, preferably is a product which displays the immunological activity of the wild type NspA protein (for instance as present on isolated Neisserial blebs). Preferably it will show at least one of the follow:
An ability to induce the production of antibodies which recognise the wild type NspA (if necessary when the NspA protein of the present disclosure is coupled to a carrier);
An ability to induce the production of antibodies that can protect against experimental infection; and
An ability to induce, when administered to an animal, the development of an immunological response that can protect against Neisserial infection such as *Neisseria meningitidis* and/or *Neisseria gonorrhoeae infection.*

The NspA protein of the present disclosure is useful in prophylatic, therapeutic and diagnostic compositions for preventing treating and diagnosing diseases caused by Neisseria, particularly *Neisseria meningitidis;* although it may also have similar applications in relation to, e.g. *Neisseria gonorrhoea* or *Neisseria lactamica.*

Standard immunological techniques may be employed with the NspA protein of the present disclosure in order to use it as an immunogen and in a vaccine. In particular, any suitable host may be injected with a pharmaceutically effective amount of the NspA protein to generate monoclonal or polyclonal anti-NspA antibodies or to induce the development of a protective immunological response against a *Neisseria* disease. Prior to administration, the NspA protein may be formulated in a suitable vehicle, and thus we provide a pharmaceutical composition comprising a pharmaceutically effective amount of one or more proteins of the present disclosure. As used herein "pharmaceutically effective amount" refers to an amount of NspA protein that elicits a sufficient titre of antibodies to treat or prevent infection. The pharmaceutical composition of the present disclosure may also comprise other antigens useful in treating or preventing disease.

The NspA protein of this disclosure may also form the basis of a diagnostic test for infection. For example, the present invention provides a method for detection of a Neisserial antigen in a biological sample containing or suspected of containing the Neisserial antigen comprising:
generating an anti-NspA antibody using the protein of the present disclosure;
isolating the biological sample from a patient;
incubating the anti-NspA antibody or a fragment thereof with the biological sample; and detecting bound antibody or bound fragment.

This disclosure also provides a method for the detection of antibody specific to NspA protein in a biological sample containing or suspected of containing said antibody comprising:
isolating the biological sample from a patient;
incubating the NspA protein of the disclosure with the biological sample; and detecting bound antigen.

This diagnostic test may take several forms including ELISA and a radioimmunoassay.

Further details on such applications are given below.

### Antibodies

The proteins of the disclosure can be used as immunogens to produce antibodies immunospecific for such proteins.

In certain preferred embodiments of the disclosure there are provided antibodies against the NspA protein of the disclosure.

Antibodies generated against the proteins of the disclosure can be obtained by administering the proteins of the disclosure, or epitope-bearing fragments of either or both, analogues of either or both, to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., Nature 256: 495-497 (1975); Kozbor et al., Immunology Today 4: 72 (1983); Cole et al., pg. 77-96 in MONOCLONAL ANTIBODIES AND CANCER THERAPY, Alan R. Liss, Inc. (1985).

Techniques for the production of single chain antibodies (U.S. Patent No. 4,946,778) can be adapted to produce single chain antibodies to proteins of this disclosure. Also, transgenic mice, or other organisms or animals, such as other mammals, may be used to express humanized antibodies immunospecific to the proteins of the disclosure.

Alternatively, phage display technology may be utilized to select antibody genes with binding activities towards a protein of the disclosure either from repertoires of PCR amplified v-genes of lymphocytes from humans screened for possessing anti-NspA or from naive libraries (McCafferty, et al., (1990), Nature 348, 552-554; Marks, et al., (1992) Biotechnology 10, 779-783). The affinity of these antibodies can also be improved by, for example, chain shuffling (Clackson et al., (1991) Nature 352: 628).

The above-described antibodies may be employed to isolate or to identify clones expressing NspA proteins of the disclosure to purify the proteins or polynucleotides by, for example, affinity chromatography.

Thus, among others, antibodies against the NspA protein of the disclosure may be employed to treat infections, particularly bacterial infections.

Preferably, the antibody or variant thereof is modified to make it less immunogenic in the individual. For example, if the individual is human the antibody may most preferably be "humanized," where the complimentarity determining region or regions of the hybridoma-derived antibody has been transplanted into a human monoclonal antibody, for example as described in Jones et al. (1986), Nature 321, 522-525 or Tempest et al., (1991) Biotechnology 9, 266-273.

A protein of the present disclosure can be administered to a recipient who then acts as a source of immune globulin, produced in response to challenge from the specific vaccine. A subject thus treated would donate plasma from which hyperimmune globulin would be obtained via conventional plasma fractionation methodology. The hyperimmune globulin would be administered to another subject in order to impart resistance against or treat Neisserial infection. Hyperimmune globulins of the disclosure are particularly useful for treatment or prevention of Neisserial disease in infants, immune compromised individuals or where treatment is required and there is no time for the individual to produce antibodies in response to vaccination.

An additional aspect of the disclosure is a pharmaceutical composition comprising a monoclonal antibody (or fragments thereof; preferably human or humanised) reactive against the pharmaceutical composition of the disclosure, which could be used to treat or prevent infection by Gram negative bacteria, preferably Neisseria, more preferably *Neisseria meningitidis* or *Neisseria gonorrhoeae* and most preferably *Neisseria meningitidis* serogroup B.

Such pharmaceutical compositions comprise monoclonal antibodies that can be whole immunoglobulins of any class e.g. IgG, IgM, IgA, IgD or IgE, chimeric antibodies or hybrid antibodies with specificity to two or more antigens of the disclosure. They may also be fragments e.g. F(ab')2, Fab', Fab, Fv and the like including hybrid fragments.

Methods of making monoclonal antibodies are well known in the art and can include the fusion of splenocytes with myeloma cells (Kohler and Milstein 1975 Nature 256; 495; Antibodies - a laboratory manual Harlow and Lane 1988). Alternatively, monoclonal Fv fragments can be obtained by screening a suitable phage display library (Vaughan TJ et al 1998 Nature Biotechnology 16; 535). Monoclonal antibodies may be humanised or part humanised by known methods.

### Vaccines

Another aspect of the disclosure relates to a method for inducing an immunological response in an individual particularly a mammal, preferably humans, which comprises inoculating the individual with NspA piotein of the present disclosure, or a fragment or variant thereof, adequate to produce antibody and/ or T cell immune response to protect (or treat) said individual from infection, particularly bacterial infection and most particularly *Neisseria meningitidis* infection. Also provided are methods whereby such immunological response slows bacterial replication.

A further aspect of the disclosure relates to an immunological composition that when introduced into an individual, preferably a human, capable of having induced within it an immunological response, induces an immunological response in such individual to a NspA protein of the present disclosure, wherein the composition comprises a recombinant NspA of the disclosure. The immunological response may be used therapeutically or prophylactically and may take the form of antibody immunity and/or cellular immunity, such as cellular immunity arising from CTL or CD4+ T cells.

A NspA protein, variant or a fragment thereof may be fused with co-protein or chemical moiety which may or may not by itself produce antibodies, but which is capable of producing a fused or modified protein which will have antigenic and/or immunogenic properties, and preferably protective properties, and optionally may stabilise the NspA, or render it easier to purify. The co-protein may act as an adjuvant in the sense of providing a generalized stimulation of the immune system of the organism receiving the protein.

Also provided by this disclosure are compositions, particularly vaccine compositions, and methods comprising the proteins of the disclosure and immunostimulatory DNA sequences, such as those described in Sato, Y. et al. Science 273: 352 (1996).

The disclosure thus also includes a vaccine formulation which comprises an immunogenic protein of the disclosure or a fragment or a variant thereof, together with a suitable carrier, such as a pharmaceutically acceptable carrier. Since the proteins may be broken down in the stomach, each is preferably administered parenterally, including, for example, administration that is subcutaneous, intramuscular, intravenous, or intradermal. Formulations suitable for parenteral administration include aqueous and non-aqueous sterile injection solutions which may contain anti-oxidants, buffers, bacteristatic compounds and solutes which render the formulation isotonic with the bodily fluid, preferably the blood, of the individual; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The formulation may also be administered mucosally, e.g. intranasally.

The vaccine formulation of the disclosure may also include adjuvant systems for enhancing the immunogenicity of the formulation. Typically aluminium phosphate or aluminium hydroxide may be used. Preferably the adjuvant system raises preferentially a TH1 type of response.

An immune response may be broadly distinguished into two extreme categories, being a humoral or cell mediated immune responses (traditionally characterised by antibody and cellular effector mechanisms of protection respectively). These categories of response have been termed TH1-type responses (cell-mediated response), and TH2-type immune responses (humoral response).

Extreme TH1-type immune responses may be characterised by the generation of antigen specific, haplotype restricted cytotoxic T lymphocytes, and natural killer cell responses. In mice TH1-type responses are often characterised by the generation of antibodies of the IgG2a subtype, whilst in the human these correspond to IgG1 type antibodies. TH2-type immune responses are characterised by the generation of a broad range of immunoglobulin isotypes including in mice IgG1, IgA, and IgM.

It can be considered that the driving force behind the development of these two types of immune responses are cytokines. High levels of TH1-type cytokines tend to favour the induction of cell mediated immune responses to the given antigen, whilst high levels of TH2-type cytokines tend to favour the induction of humoral immune responses to the antigen.

The distinction of TH1 and TH2-type immune responses is not absolute. In reality an individual will support an immune response which is described as being predominantly TH1 or predominantly TH2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173*).* Traditionally, TH1-type responses are associated with the production of the INF-γ and IL-2 cytokines by T-lymphocytes. Other cytokines often directly associated with the induction of TH1-type immune responses are not produced by T-cells, such as IL-12. In contrast, TH2- type responses are associated with the secretion of IL-4, IL-5, IL-6 and IL-13.

It is known that certain vaccine adjuvants are particularly suited to the stimulation of either TH1 or TH2 - type cytokine responses. Traditionally the best indicators of the TH1:TH2 balance of the immune response after a vaccination or infection includes direct measurement of the production of TH1 or TH2 cytokines by T lymphocytes *in vitro* after restimulation with antigen, and/or the measurement of the IgG1:IgG2a ratio of antigen specific antibody responses.

Thus, a TH1-type adjuvant is one which preferentially stimulates isolated T-cell populations to produce high levels of TH1-type cytokines when re-stimulated with antigen *in vitro,* and promotes development of both CD8+ cytotoxic T lymphocytes and antigen specific immunoglobulin responses associated with TH1-type isotype.

Adjuvants which are capable of preferential stimulation of the TH1 cell response are described in International Patent Application No. WO 94/00153 and WO 95/17209. 3 De-O-acylated monophosphoryl lipid A (3D-MPL) is one such adjuvant, and is preferred. This is known from GB 2220211 (Ribi). Chemically it is a mixture of 3 De-O-acylated monophosphoryl lipid A with 4, 5 or 6 acylated chains and is manufactured by Ribi Immunochem, Montana. A preferred form of 3 De-O-acylated monophosphoryl lipid A is disclosed in European Patent 0 689 454 B1 (SmithKline Beecham Biologicals SA). Alternatively, other non-toxic derivatives of LPS may be used.

Preferably, the particles of 3D-MPL are small enough to be sterile filtered through a 0.22micron membrane (European Patent number 0 689 454). 3D-MPL will be present in the range of 10µg - 100µg preferably 25-50µg per dose wherein the antigen will typically be present in a range 2-50µg per dose.

Another preferred adjuvant comprises QS21, an Hplc purified non-toxic fraction derived from the bark of Quillaja Saponaria Molina. Optionally this may be admixed with 3 De-O-acylated monophosphoryl lipid A (3D-MPL), or non-toxic LPS derivative, optionally together with a carrier.

The method of production of QS21 is disclosed in US patent No. 5,057,540.

Non-reactogenic adjuvant formulations containing QS21 have been described previously (WO 96/33739). Such formulations comprising QS21 and cholesterol have been shown to be successful TH1 stimulating adjuvants when formulated together with an antigen.

Further adjuvants which are preferential stimulators of TH1 cell response include immunomodulatory oligonucleotides, for example unmethylated CpG sequences as disclosed in WO 96/02555.

Combinations of different TH1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of 1 : 10 to 10 : 1; preferably 1:5 to 5 : 1 and often substantially 1 : 1. The preferred range for optimal synergy is 2.5 : 1 to 1 : 1 3D-MPL: QS21.

Preferably a carrier is also present in the vaccine composition according to the disclosure. The carrier may be an oil in water emulsion, or an aluminium salt, such as aluminium phosphate or aluminium hydroxide.

A preferred oil-in-water emulsion comprises a metabolisible oil, such as squalene, alpha tocopherol and Tween 80. In a particularly preferred aspect the antigens in the vaccine composition according to the disclosure are combined with QS21 and 3D-MPL in such an emulsion. Additionally the oil in water emulsion may contain span 85 and/or lecithin and/or tricaprylin.

Typically for human administration QS21 and 3D-MPL will be present in a vaccine in the range of 1µg - 200µg, such as 10-100µg, preferably 10µg - 50µg per dose. Typically the oil in water will comprise from 2 to 10% squalene, from 2 to 10% alpha tocopherol and from 0.3 to 3% tween 80. Preferably the ratio of squalene: alpha tocopherol is equal to or less than 1 as this provides a more stable emulsion. Span 85 may also be present at a level of 1%. In some cases it may be advantageous that the vaccines of the present disclosure will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

The present disclosure also provides a polyvalent vaccine composition comprising a vaccine formulation of the disclosure in combination with other antigens, in particular antigens useful for treating cancers, autoimmune diseases and related conditions. Such a polyvalent vaccine composition may include a TH-1 inducing adjuvant as hereinbefore described.

### Subunit composition

The composition of the present disclosure is preferably in the form of a subunit composition. Subunit compositions are compositions in which the components have been isolated and purified to at least 50%, preferably at least 60%, 70%, 80%, 90% pure before mixing the components to form the antigenic composition.

Subunit compositions may comprise aqueous solutions of water soluble proteins. They may comprise detergent, preferably non-ionic, zwitterionic or ionic detergent in order to solubilise hydrophobic portions of the antigens. They may comprise lipids so that liposome structures could be formed, allowing presentation of antigens with a structure that spans a lipid membrane. Further details on compositions is given below.

The subunit composition of the invention may also comprise at least one further antigen which may be soluble, or may be presented on the outer membrane of a bleb (OMV) (thus making a mixed subunit/bleb composition).

Neisserial infections progress through several different stages. For example, the meningococcal life cycle involve nasopharyngeal colonisation, mucosal attachment, crossing into the bloodstream, multiplication in the blood, induction of toxic shock, crossing the blood/brain barrier and multiplication in the cerebrospinal fluid and/or the meninges. Different molecules on the surface of the bacterium will be involved in different steps of the infection cycle. By targeting the immune response against an effective amount of a combination of particular antigens, involved in different processes of Neisserial infection, a Neisserial vaccine with surprisingly high efficacy can be achieved.

In particular, combinations of certain Neisserial antigens from different classes with the NspA protein of the disclosure can elicit an immune response which protects against multiple stages of infection. Such combinations of antigens can surprisingly lead to synergistically improved vaccine efficacy against Neisserial infection where more that one function of the bacterium is targeted by the immune response in an optimal fashion. Some of the further antigens which can be included are involved in adhesion to host cells, some are involved in iron acquisition, some are autotransporters and some are toxins. As NspA is an adhesin, it is preferred that it is mixed with an iron acquisition protein, autotransporter or toxin, or an iron acquisition protein and autotransporter, or an iron acquisition protein and a toxin, or an autotransporter and a toxin, or an iron acquisition protein and autotransporter and toxin.

An additional advantage of the disclosure is that the combination of the antigens of the disclosure from different families of proteins in an immunogenic composition will enable protection against a wider range of strains.

The present disclosure also relates to a combination of NspA of the disclosure with one or more (e.g. two, three, four, five, six, seven, eight, nine or ten) different Neisserial proteins. Such additional proteins may be selected from the group consisting of: FhaB, Hsf, NadA, Hap, FrpA, FrpB, FrpC, LPS immunotype L2, LPS immunotype L3, TbpA, TbpB, LbpA, LbpB, TspA, TspB, PilQ, omp85 and PldA.

The disclosure also relates to immunogenic compositions comprising a plurality of proteins selected from at least two different categories of protein, having different functions within Neisseria. Examples of such categories of proteins are adhesins, autotransporter proteins, toxins and Fe acquisition proteins. The vaccine combinations of the disclosure show surprising improvement in vaccine efficacy against homologous Neisserial strains (strains from which the antigens are derived) and preferably also against heterologous Neisserial strains.

In particular, the disclosure provides immunogenic compositions that comprise at least two, three, four five, six, seven, eight, nine or ten different Neisseria antigens (one of which is the NspA of the disclosure; classified as either an adhesin or outer membrane protein) selected from at least one, two, three, four or five different groups of proteins selected from the following:
- at least one Neisserial adhesin selected from the group consisting of FhaB, PilC, Hsf, Hap, MafA, MafB, Omp26, NMB 0315, NMB 0995, NMB 1119 and NadA;
- at least one Neisserial autotransporter selected from the group consisting of Hsf, Hap, IgA protease, AspA, and NadA;
- at least one Neisserial toxin selected from the group consisting of FrpA, FrpC, FrpA/C, VapD, NM-ADPRT and either or both of LPS immunotype L2 and LPS immunotype L3;
- at least one Neisserial Fe acquisition protein selected from the group consisting of TbpA, TbpB, LbpA, LbpB, HpuA, HpuB, Lipo28 (GNA2132), Sibp, NMB0964, NMB0293, FbpA, Bcp, BfrA, BfrB and P2086 (XthA); and
- at least one Neisserial membrane-associated protein, preferably outer membrane protein, particularly integral outer membrane protein, selected from the group consisting of PilQ, OMP85, FhaC, TbpA, LbpA, TspA, TspB, TdfH, PorB, MltA, HpuB, HimD, HisD, GNA1870, OstA, HlpA (GNA1946) , NMB 1124, NMB 1162, NMB 1220, NMB 1313, NMB 1953, HtrA, and PldA (Omp1A).
preferably:
at least one Neisserial adhesin selected from the group consisting of FhaB, Hsf and NadA;
at least one Neisserial autotransporter selected from the group consisting of Hsf, Hap and NadA;
at least one Neisserial toxin selected from the group consisting of FrpA, FrpC, and either or both of LPS immunotype L2 and LPS immunotype L3;
at least one Neisserial Fe acquisition protein selected from the group consisting of TbpA, TbpB, LbpA and LbpB; and
at least one Neisserial outer membrane protein selected from the group consisting of TspA, TspB, PilQ, OMP85, and PldA.

Preferably the first four (and most preferably all five) groups of antigen are represented in the immunogenic composition of the disclosure.

As previously mentioned where a protein is specifically mentioned herein, it is preferably a reference to a native, full-length protein but it may also encompass antigenic fragments thereof (particularly in the context of subunit vaccines). These are fragments containing or comprising at least 10 amino acids, preferably 20 amino acids, more preferably 30 amino acids, more preferably 40 amino acids or most preferably 50 amino acids, taken contiguously from the amino acid sequence of the protein. In addition, antigenic fragments denotes fragments that are immunologically reactive with antibodies generated against the Neisserial proteins or with antibodies generated by infection of a mammalian host with Neisseria. Antigenic fragments also includes fragments that when administered at an effective dose, elicit a protective immune response against Neisserial infection, more preferably it is protective against *N. meningitidis* and/or *N. gonorrhoeae* infection, most preferably it is protective against *N. meningitidis* serogroup B infection.

Also included in the disclosure are recombinant fusion proteins of Neisserial proteins of the disclosure, or fragments thereof. These may combine different Neisserial proteins or fragments thereof in the same protein. Alternatively, the disclosure also includes individual fusion proteins of Neisserial proteins or fragments thereof, as a fusion protein with heterologous sequences such as a provider of T-cell epitopes, or viral surface proteins such as influenza virus haemagglutinin, tetanus toxoid, diphtheria toxoid, CRM197.

### Compositions, kits and administration

As previously mentioned the disclosure provides compositions comprising a NspA protein for administration to a cell or to a multicellular organism.

An immunogenic composition is a composition comprising at least one antigen which is capable of generating an immune response when administered to a host. Preferably, such immunogenic preparations are capable of generating a protective immune response against Neisserial, preferably *Neisseria meningitidis and*/*or Neisseria gonorrhoeae* infection.

The disclosure also relates to compositions comprising a protein discussed herein or their agonists or antagonists. The proteins of the disclosure may be employed in combination with a non-sterile or sterile carrier or carriers for use with cells, tissues or organisms, such as a pharmaceutical carrier suitable for administration to an individual. Such compositions comprise, for instance, a media additive or a therapeutically effective amount of a protein of the disclosure and a pharmaceutically acceptable carrier or excipient. Such carriers may include, but are not limited to, saline, buffered saline, dextrose, water, glycerol, ethanol and combinations thereof. The formulation should suit the mode of administration. The invention further relates to diagnostic and pharmaceutical packs and kits comprising one or more containers filled with one or more of the ingredients of the aforementioned compositions of the disclosure.

Proteins and other compounds of the disclosure may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

In therapy or as a prophylactic, the active agent may be administered to an individual as an injectable composition, for example as a sterile aqueous dispersion, preferably isotonic.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a protein or other compounds of the present disclosure can be formulated in an enteric or an encapsulated formulation, oral administration may also be possible. Administration of these compounds may also be topical and/or localized, in the form of salves, pastes, gels, solutions, powders and the like.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this disclosure.

The dosage range required depends on the choice of peptide, the route of administration, the nature of the formulation, the nature of the subject's condition, and the judgement of the attending practitioner. Suitable dosages, however, are in the range of 0.1-100 µg/kg of subject.

A vaccine composition is conveniently in injectable form. Conventional adjuvants may be employed to enhance the immune response. A suitable unit dose for vaccination is 0.5-5 microgram/kg of antigen, and such dose is preferably administered 1-3 times and with an interval of 1-3 weeks. With the indicated dose range, no adverse toxicological effects will be observed with the compounds of the disclosure which would preclude their administration to suitable individuals.

Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Preferred features and embodiment of the present invention will now be described further with reference to the following Examples:

### Example 1 - Preparation of native NspA (Neisserial Surface Protein A)

Genomic DNA of *Neisseria meningitidis* strain H44/76 was prepared using the Quiagen genomic preparation kit The part of the NspA gene encoding the mature NspA protein, i.e. without the 19 amino acid signal peptide, was PCR amplified from genomic H44/76 DNA using the primer pair 5' gctacatatggaaggcgcatccggcttttacg and 5' gctaggatcctcagaatttgacgcgcacaccgg.

The resultant PCR product was cloned into pCRII-TOPO (Invitrogen), digested with *Nde*I and *Bam*HI and ligated into pET11a (Novagen). The resultant plasmid pET11a-NspA was transformed into BL21-DE3 cells (Novagen). Five liters of these cells were grown at 37°C in Luria Broth containing 100 ug/ml ampicillin. After reaching an OD600 of 0.6, 1 mM of isopropyl-thio-β-D-galactopyranoside (IPTG) was added for 2 hours (alternatively 4 additional hours with 0.1 mM IPTG was used). Cells were harvested and washed with 600 ml of 0,9% (w/v) NaCl. NspA was present as inclusion bodies. The cell pellet was resuspended in 100 ml ice-cold TE buffer (50 mM Tris/HCl + 40 mM EDTA pH 8.0). Twenty-five grams of sucrose and 20 mg lysozyme was added for 30 minutes with shaking. A hundred mls of TE was added and shaking was continued for another 30 minutes. Then 100 ml fractions were sonicated (Branson sonifier), 4 mls of 10% Brij were added with another round of sonication. Suspensions were spun 30 minutes at 4000 rpm. The pellet containing inclusion bodies was washed once with TE buffer, resuspended in 40 mls of 10 mM Tris/HCl pH 8.3, spun in aliquots (8000 rpm Eppendorf centrifuge) and frozen at -20 C.

Inclusion bodies were solubilized in 20 mM Tris/HCl + 0.1 M glycine + 8 M urea pH 8.0.

Solubilized, denatured NspA protein was refolded by dilution (1:20) into 20 mM ethanolamine containing 0.5% purified SB-12 (3-dimethyldodecylammoniopropanesulfonate, Fluka - DodMe2NprSO3). SB-12 was purified by passing a concentrated solution of the detergent in methanol/chloroform (1:1) over an Al₂O₃ column. Alternatively, a 5-fold dilution into 20 mM ethanolamine, pH 11, containing 1% (w/v) purified SB-12 (3-dimethyldodecylammoniopropanesulfonate, Fluka) was carried out. The solution was left stirring overnight at room temperature.

Refolding was evaluated by semi-native SDS-PAGE (i.e. running with as little as necessary SDS, low amperage, at 4C), which reveals refolded NspA as a slower running form compared to denatured NspA (5 min boiling with 1% SDS). This is consistent with its running behaviour when present in native cell envelopes as reported in the literature. Judged by silver staining of the gels, 100% refolding efficiency was achieved. Gels usually contained 14% acrylamide, with no SDS in the gel and only 0.03% SDS in the 3X sample buffer (additionally comprising 0.1M Tris pH 6.8, 15.4% glycerol, 7.7% β-mercaptoethanol, and 0.008% (w/v) bromophenol blue), and were not heated before electrophoresis.

To further purify the folded NspA, the mixture was loaded onto a 1 ml mono S column (Pharmacia), pre-equilibrated with 10 mM DodMe2NprSO3, 20 mM Tris-HCl pH 8.5 (buffer A). Prior to loading, the pH of the protein sample was adjusted to pH 8.5 using 1 M HCl. The column was washed with buffer A, and proteins were eluted with a linear gradient of 0-500 mM NaCl in buffer A, total volume 50 ml.

Although OMPs are generally heat modifiable, i.e. folded monomers run faster in SDS-PAGE gels than heat-denatured proteins, in contrast we observed that NspA migrated slower in the gel after refolding. The denatured protein migrated at a mass of ∼18 kDa, whereas the folded protein migrated at 22 kDa, which agrees well with the electrophoretic mobility of native NspA isolated from neisserial membranes (Moe et al. 1999 I&I 67:5664). Analytical ultracentrifugation and chemical cross-linking experiments confirmed that refolded NspA was a monomer (i.e. did not dimerize) in a detergent-containing solution, as confirmed by its crystal structure (see below).

### Example 2- Evidence of the properly folded structure of refolded NspA

In addition to the above evidence that refolded NspA migrated on a gel in the same way as native NspA isolated from neisserial membranes, 2 further indicators of proper refolding were carried out.

Refolded NspA was injected into mice, and the resulting sera obtained was able to recognise native (non-denatured) NspA in an ELISA test.

Refolded NspA was also used to generate crystals of high quality from which the crystal structure of folded Neisserial NspA was solved (Vandeputte-Rutten et al. 2003 JBC 278:24825).

### Example 3 - Serum bactericidal activity of antibodies against NspA in combination with TbpA and Hsf

*N. meningitidis* strain H66/76 in which the expression of PorA and the synthesis of capsular polysaccharides were switched off (see WO 01/09350), was used as the background strain for up-regulating the expression of TbpA and Hsf, or of NspA using the procedures described in WO 01/09350. Outer membrane vesicles were prepared from the strains. TbpA may be upregulated genetically or by growing the production strain in low iron conditions.

The outer membrane vesicle preparations were adsorbed onto Al(OH)3 and injected into mice on days 0, 21 and 28. On day 42, the mice were bled and sera prepared. The sera against TbpA and Hsf up-regulated OMVs were mixed with sera from mice vaccinated with OMVs containing up-regulated NspA and serum bactericidal assays were performed as follows.

The serum bactericidal activity of antisera from the mice inoculated with OMVs were compared in assays using either the homologous strain H44/76 or the heterologous strain Cu385. The serum bactericidal assay has been shown to show good correlation with protection and is therefore a good indication of how effective a candidate composition will be in eliciting a protective immune response.

*Neisseria meningitidis* serogroup B wild type strains (H44/76 strain =B:15 P1.7,16 L3,7,9 and CU385 strain =B: 4 P1.19,15 L3,7,9) were cultured overnight on MH + 1% Polyvitex + 1% horse serum Petri dishes at 37°C + 5% CO2. They were subcultured for 3 hours in a liquid TSB medium supplemented with 50 µM of Desferal (Iron chelator) at 37°C under shaking to reach an optical density of approximately 0.5 at 470 nm.

Pooled or individual sera were inactivated for 40 min at 56°C. Serum samples were diluted 1/100 in HBSS-BSA 0.3% and then serially diluted two fold (8 dilutions) in a volume of 50 µl in round bottom microplates.

Bacteria, at the appropriate OD, were diluted in HBSS-BSA 0.3% to yield 1.3 10e4 CFU per ml. 37.5 µl of this dilution was added to the serum dilutions and microplates were incubated for 15 minutes at 37°C under shaking. Then, 12.5 µl of rabbit complement were added to each well. After 1 hour of incubation at 37°C and under shaking, the microplates were placed on ice to stop the killing.

Using the tilt method, 20µl of each well were plated on MH + 1% Polyvitex + 1% horse serum Petri dishes and incubated overnight at 37°C +CO2. The CFU's were counted and the percent of killing calculated. The serum bactericidal titer is the last dilution of serum yielding ≥ 50% killing.

Mice are thought to produce a significant level of bactericidal antibodies if titres are greater than 1/100, particularly in experiments using the heterologous strain.

### Results

Results are shown in the table below. In assays using the homologous H44/76 stain, the addition of antibodies against NspA, did not produce a serum bactericidal titre higher than that produced using antibodies against TbpA and Hsf alone.

However, the addition of antibodies against NspA was advantageous in serum bactericidal assays using a heterologous strain. Antibodies against NspA were effective at increasing the serum bactericidal titre against the CU385 strain.

| | Serum Bactericidal Titre | |
|---|---|---|
| Antisera Mix | H44/76 | CU385 |
| antiTbpA-Hsf and nonimmune sera | 5378 | 2141 |
| anti-TbpA-Hsf and anti-NspA | 4738 | 2518 |

NspA when combined with TbpA and/or Hsf in an effective amount (upregulated) is able to improve the cross-bactericidal character of sera generated against the combined vaccine.

Although the invention has been described in connection with specific preferred embodiments, it should be understood that the invention as claimed should not be unduly limited to such specific embodiments.

## Claims

1. A method for refolding an NspA protein comprising contacting the NspA protein with an alkaline refolding buffer comprising 3-dimethyldodecylammoniopropanesulfonate (SB-12).

2. A method according to claim 1 wherein the refolding buffer comprises ethanolamine and SB-12.

3. A method according to claim 2 wherein the ethanolamine is 20mM ethanolamine.

4. A method according to any one of claims 1 to 3 wherein the refolding buffer has pH11.

5. A method according to any one of claims 1 to 4 wherein the SB-12 is 0.2% SB-12.

6. A method according to any one of claims 1 to 4 wherein the SB-12 is 0.5% SB-12.

7. A method according to any one of claims 1 to 6 wherein the SB-12 is purified.

8. A method according to claim 7 wherein the SB-12 is purified by passing it over an Al₂O₃ column.

9. A method comprising the following steps:
a. expressing an NspA protein in a host cell;
b. breaking the host cell to obtain an inclusion body comprising the NspA protein;
c. washing the inclusion body;
d. solubilisation of at least part of the inclusion body and the NspA protein;
e. contacting the solubilised NspA protein with the refolding buffer of claims 1-8; and
f. removing the refolding buffer from the NspA protein.

## Patentansprüche

1. Verfahren zur Neufaltung eines NspA-Proteins, welches das Kontaktieren des NspA-Proteins mit einem alkalischen Neufaltungspuffer, welcher 3-Dimethyldodecylammoniumpropansulfonat (SB-12) enthält, umfasst.

2. Verfahren gemäß Anspruch 1, wobei der Neufaltungspuffer Ethanolamin und SB-12 enthält.

3. Verfahren gemäß Anspruch 2, wobei das Ethanolamin 20 mM Ethanolamin ist.

4. Verfahren gemäß einem der Ansprüche 1 bis 3, wobei der Neufaltungspuffer pH 11 hat.

5. Verfahren gemäß einem der Ansprüche 1 bis 4, wobei das SB-12 0,2% SB-12 ist.

6. Verfahren gemäß einem der Ansprüche 1 bis 4, wobei das SB-12 0,5% SB-12 ist.

7. Verfahren gemäß einem der Ansprüche 1 bis 6, wobei das SB-12 aufgereinigt ist.

8. Verfahren gemäß Anspruch 7, wobei das SB-12 aufgereinigt ist, indem es über eine Al₂O₃-Säule laufen gelassen wird.

9. Verfahren, welches die folgenden Schritte umfasst:
a. Exprimieren eines NspA-Proteins in einer Wirtszelle;
b. Öffnen der Wirtszelle, um einen Einschlusskörper, welcher das NspA-Protein enthält, zu erhalten;
c. Waschen des Einschlusskörpers;
d. Solubilisieren von mindestens einem Teil des Einschlusskörpers und des NspA-Proteins;
e. Kontaktieren des solubilisierten NspA-Proteins mit dem Neufaltungspuffer gemäß den Ansprüchen 1-8; und
f. Entfernen des Neufaltungspuffers vom NspA-Protein.

## Revendications

1. Procédé de repliement d'une protéine NspA comprenant la mise en contact de la protéine NspA avec un tampon de repliement alcalin comprenant le 3-diméthyldodécylammoniopropane sulfonate (SB-12).

2. Procédé selon la revendication 1, dans lequel le tampon de repliement comprend de l'éthanolamine et du SB-12.

3. Procédé selon la revendication 2, dans lequel l'éthanolamine est de l'éthanolamine 20 mM.

4. Procédé selon l'une quelconque des revendications 1 à 3, dans lequel le tampon de repliement a un pH de 11.

5. Procédé selon l'une quelconque des revendications 1 à 4, dans lequel le SB-12 est du SB-12 à 0,2 %.

6. Procédé selon l'une quelconque des revendications 1 à 4, dans lequel le SB-12 est du SB-12 à 0,5 %.

7. Procédé selon l'une quelconque des revendications 1 à 6, dans lequel le SB-12 est purifié.

8. Procédé selon la revendication 7, dans lequel le SB-12 est purifié en passant sur une colonne d'Al₂O₃.

9. Procédé comprenant les étapes suivantes :
a. l'expression d'une protéine NspA dans une cellule hôte ;
b. la rupture de la cellule hôte pour obtenir un corps d'inclusion comprenant la protéine NspA ;
c. le lavage du corps d'inclusion ;
d. la solubilisation d'au moins une partie du corps d'inclusion et de la protéine NspA ;
e. la mise en contact de la protéine NspA solubilisée avec le tampon de repliement selon les revendications 1 à 8 ; et
f. le retrait du tampon de repliement de la protéine NspA.
